# EUROPEAN PATENT APPLICATION

(11) **EP 4 810 538 A1**
(43) Date of publication of application: **23.09.2026**
(21) Application number: 23958608.4
(22) Date of filing: 17.11.2023
(51) Int. Cl.: C08G 63/685, C08G 63/08, C08G 18/32, C08G 18/42, C08L 75/04, C08K 5/3475, C07D 249/20

(54) **ULTRAVIOLET LIGHT ABSORBING SUBSTANCE AS WELL AS PREPARATION METHOD THEREFOR AND USE THEREOF**

(71) Applicant: Chitec Technology Co., Ltd., Taipei City 110 (TW)
(72) Inventor: WU, Huang-min, Taipei City Taiwan (TW); CHEN, Si-yuan, Taipei City Taiwan (TW); CHENG, Ching-hao, Taipei City Taiwan (TW); HUANG, Yi-shuo, Taipei City Taiwan (TW); CHANG, Wei-chun, Taipei City Taiwan (TW); WU, Chi-feng, Taipei City Taiwan (TW); LUO, De-shun, Taipei City Taiwan (TW); SUNG, Wei-cheng, Taipei City Taiwan (TW)
(74) Representative: dompatent
(86) International application number: PCT/CN2023/132230
(87) International publication number: WO 2025/102334

(57) **Abstract**

The present invention provides a substance represented by formula I, wherein R1 is H or Cl, A is C₂ to C₅ alkylene, B is C₂ to C₅ alkylene, m+n is an integer from 2 to 120, and none of m and n is 0. The substance can be used as an ultraviolet light absorber.

## Description

### Field of the Invention

The present invention relates to a substance that can be used for ultraviolet light absorption, in particular to a substance having a benzotriazole group and a polyol structure. The substance of the present invention can be used as an ultraviolet light absorber and can be applied to various polymer materials.

### Descriptions of the Related Art

Polymer materials are widely used in various products due to their diverse structures and properties. However, some polymer materials are susceptible to degradation upon exposure to ultraviolet light. Therefore, it has been conventionally known to incorporate ultraviolet light absorbers (UV absorbers, UVA) into polymer materials to provide resistance against ultraviolet light.

Taking polyurethane as an example, it is an important class of polymers formed by polymerization of polyols and isocyanates. By adjusting the ratio of the raw materials, materials having desired mechanical properties, such as abrasion resistance, temperature resistance, flexibility, and elongation, can be produced, for example, coatings, elastomers, foams, adhesives, and sealants. However, polyurethane materials are prone to degradation upon exposure to ultraviolet light, and such deterioration is particularly rapid under strong outdoor light conditions. In order to prevent degradation caused by ultraviolet light, ultraviolet light absorbers may be physically incorporated into polyurethane, among which benzotriazole (BTZ)-type ultraviolet light absorbers are the most widely used.

However, ultraviolet light absorbers that are physically incorporated tend to undergo migration in polyurethane materials, which in turn may cause blooming of the polyurethane materials or impair the surface properties thereof. For example, the surface of the polyurethane materials may become tacky, or even the applied products may undergo fading. Therefore, how to improve the compatibility of ultraviolet light absorbers in polyurethane materials so as to avoid or reduce migration has become an important issue in the development of ultraviolet light absorbers. In general, the compatibility of ultraviolet light absorbers in polyurethane materials may be improved by the following two approaches so as to reduce or avoid migration of the ultraviolet light absorbers.

A first approach is to increase the molecular weight of the ultraviolet light absorber, as disclosed in US 4,853,471 and US 7,381,762, wherein the migration rate of the ultraviolet light absorber molecules in polyurethane materials is reduced by increasing the molecular weight of the molecule of ultraviolet light absorber. However, this approach can only slow down the migration rate and cannot effectively prevent migration. Moreover, increasing the molecular weight of the ultraviolet light absorber correspondingly reduces the effective content thereof, thereby requiring an increased amount of the ultraviolet light absorber to achieve a comparable ultraviolet light resistance.

A second approach is to synthesize the ultraviolet light absorber as a reactive ultraviolet light absorber, wherein hydroxyl groups contained therein participate in the polymerization reaction during the synthesis of polyurethane, such that the ultraviolet light absorber is incorporated into the polyurethane structure via chemical bonding. Examples of such reactive ultraviolet light absorbers include those disclosed in US 5,459,222 and TW I638039.

In terms of performance, the second approach can more effectively address the migration problem of ultraviolet light absorbers. However, the reactive ultraviolet light absorbers disclosed in the prior art still fail to simultaneously exhibit advantages such as low-temperature operability (being flowable and readily soluble/dispersible at low temperatures of 40°C to 50°C), good thermal stability, and excellent ultraviolet light resistance.

### Summary of the Invention

In view of the foregoing technical problems, the present invention provides a substance capable of absorbing ultraviolet light, which has a benzotriazole group and a polyol structure. The substance can be directly bonded to a polymer structure via chemical bonding, thereby solving the migration problem of ultraviolet light absorbers. In addition, the substance simultaneously exhibits advantages such as low-temperature operability (being flowable and readily soluble/dispersible at low temperatures of 40°C to 50°C, for example, being readily soluble and dispersible in polyols), good thermal stability, and excellent ultraviolet light resistance.

Therefore, an objective of the present application is to provide a substance represented by Formula I, 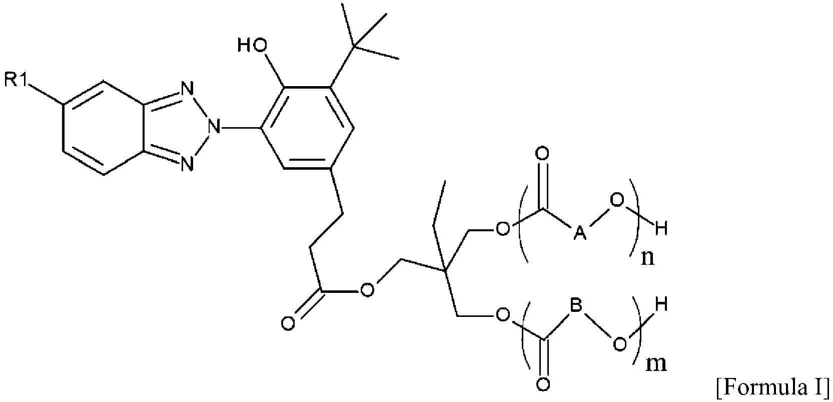 wherein,
R1 is H or Cl;
A is C₂ to C₅ alkylene;
B is C₂ to C₅ alkylene; and
m+n is an integer from 2 to 120, with neither m nor n being 0.

In some embodiments of the present invention, each of A and B is independently C₅ alkylene.

In some embodiments of the present invention, each of m and n is independently an integer from 1 to 50.

Another object of the present invention is to provide a use of the substance of Formula I as an ultraviolet light absorber.

Still another object of the present invention is to provide a polymer precursor composition comprising: a polymerizable monomer; and the substance of Formula I.

In some embodiments of the present invention, the polymerizable monomer comprises a polyol and a polyisocyanate.

In some embodiments of the present invention, the polymer precursor composition further comprises at least one additive selected from the group consisting of solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, biocides, and combinations thereof.

Still another object of the present invention is to provide a polymer comprising a structure derived from the substance of Formula I.

In some embodiments of the present invention, the polymer is selected from the group consisting of polyurethane, polyester, polycarbonate, epoxy resin, amino resin, polyamide, polyimide, liquid crystal polymer, polyoxymethylene, polysiloxane, polymethacrylate copolymer, polyacrylate copolymer, and composites thereof.

Still another object of the present invention is to provide an article capable of resisting ultraviolet light, wherein the substance of Formula I is used as an ultraviolet light absorber. In addition to the substance of Formula I, the article may further comprise other conventional ultraviolet light absorbers.

In some embodiments of the present invention, the article is selected from the group consisting of plastics, coatings, inks, displays, lighting devices, optical films, optical lenses, goggles, eyeglasses, contact lenses, textiles, pressure-sensitive adhesives, and sunscreen products.

In some embodiments of the present invention, the article further comprises at least one additive selected from the group consisting of solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, biocides, and combinations thereof.

Still another object of the present invention is to provide a method for preparing an article capable of resisting ultraviolet light, which comprises using the substance of Formula I in the article.

Still another object of the present invention is to provide a method for preparing the substance of Formula I, which comprises reacting a compound represented by Formula II with a C₃ to C₆ lactone compound in the presence of a ring-opening polymerization catalyst, wherein R1 is H or Cl.

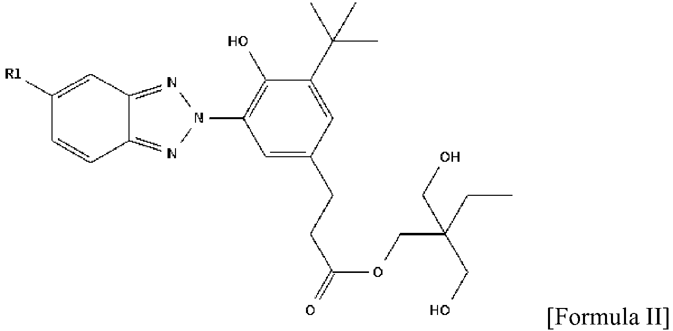

In some embodiments of the present invention, the ring-opening polymerization catalyst is diphenyl phosphate.

In some embodiments of the present invention, the C₃ to C₆ lactone compound is selected from the group consisting of β-propiolactone, γ-butyrolactone, δ-valerolactone, ε-caprolactone, and combinations thereof.

To render the above objectives, technical features and advantages of the present invention more apparent, the present invention will be described in detail with reference to some embodiments hereinafter.

### Description of the Drawings

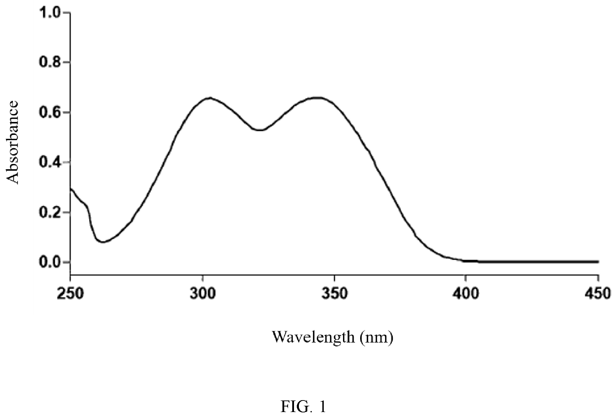

FIG. 1 is an ultraviolet absorption spectrum of the substance prepared in Synthesis Example 1.

### Description of the Preferred Embodiments

Hereinafter, some embodiments of the present application will be described in detail. However, the present application may be embodied in various embodiments and should not be limited to the embodiments described in the specification.

Unless additionally explained, the expressions "a," "the," or the like, as recited in the specification and the claims, should include both the singular and plural forms.

The inventors have found that a substance having a benzotriazole group and a polyol structure can be synthesized by a simple method. In addition to being capable of solving the migration problem of conventional ultraviolet light absorbers, the substance further simultaneously exhibits advantages such as low-temperature operability (being flowable and readily soluble/dispersible at low temperatures of 40°C to 50°C, for example, being readily soluble and dispersible in polyols), good thermal stability, and excellent ultraviolet light resistance. Accordingly, the substance is suitable for use in various fields requiring resistance to ultraviolet light. The substance of the present invention, as well as the preparation method and applications thereof, are described in detail below.

### 1. Substance of Formula I

In the present invention, the substance represented by Formula I has the following structure: 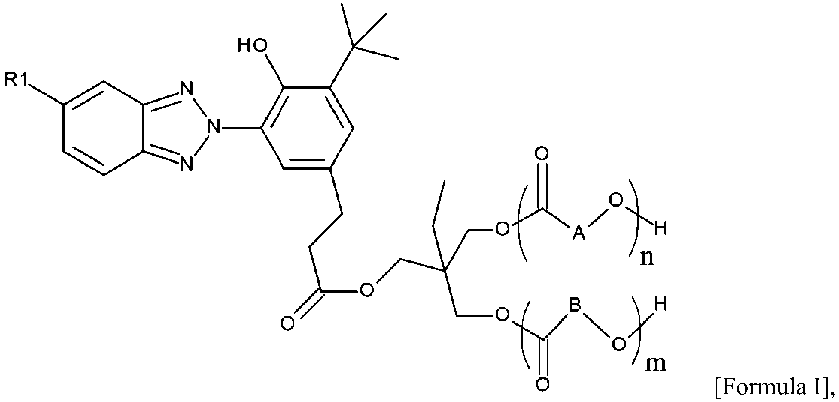 in formula I, R1 is H or Cl; A is C₂ to C₅ alkylene; B is C₂ to C₅ alkylene; and m+n is an integer from 2 to 120, with neither m nor n being 0.

The C₂ to C₅ alkylene refers to a divalent group formed by removing one hydrogen atom from each of two carbon atoms of an alkyl group. In the present invention, A and B are each independently C₂ to C₅ alkylene, and in embodiments where n is greater than 1, each A may be the same or different, and in embodiments where m is greater than 1, each B may be the same or different. In some embodiments of the present invention, the C₂ to C₅ alkylene is a linear C₂ to C₅ alkylene, examples of which include ethylene, propylene, butylene, and pentylene. In some embodiments of the present invention, each of A and B is independently C₅ alkylene, preferably linear C5 alkylene, i.e., pentylene.
m and n respectively represent the number of structural units within the parentheses. In the present invention, m+n is an integer from 2 to 120, and neither m nor n is 0. Preferably, m+n is an integer from 10 to 100, for example, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100, or within a range between any two of the values described herein.

As will be described in detail below with reference to the synthesis examples, the substance having the structure of Formula I of the present invention can be prepared by subjecting a compound represented by Formula II and a C₃ to C₆ lactone compound to a polymerization reaction in the presence of a ring-opening polymerization catalyst. Under the reaction mechanism, it is expected that m and n in Formula I are approximately the same. Therefore, in some embodiments of the present invention, each of m and n is independently an integer from 1 to 60, preferably each independently an integer from 1 to 50, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, or within a range between any two of the values described herein.

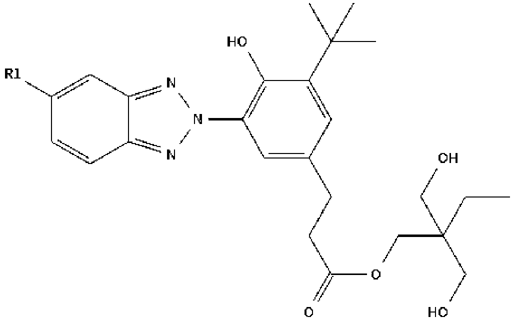

The substance represented by Formula I of the present invention has a benzotriazole group (i.e., 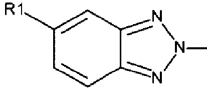 ) and a polyol structure, in particular a long-chain polyol structure. Without being bound by theory, it is believed that such structures provide at least the following advantages: good dispersibility at low operating temperatures (for example, at 50°C); excellent hydrolysis resistance; excellent solubility in alkaline conditions and in alcohols, enabling the substance to be dissolved in alcohols at low temperatures; and since polyols are key raw materials for forming certain polymers (such as polyurethanes), the compound is particularly useful in such polymers; as well as excellent thermal stability and ultraviolet resistance.

### 2. Use of the Substance of Formula I

### 2.1. Use as an Ultraviolet Light Absorber

The substance represented by Formula I can absorb ultraviolet light and is therefore suitable for use as an ultraviolet light absorber in various fields requiring resistance to ultraviolet light.

### 2.2. Polymer Precursor Composition

The substance represented by Formula I can be applied in polymer materials to provide resistance to ultraviolet light. Accordingly, the present invention also provides a polymer precursor composition comprising a polymerizable monomer and the substance represented by Formula I.

The term "polymerizable monomer" refers to any compound capable of reacting with each other and preferably capable of reacting with the substance represented by Formula I to form a polymer. Preferably, the polymerizable monomer can react with the substance represented by Formula I such that the resulting polymer comprises a structure derived from the substance represented by Formula I.

In some embodiments of the present invention, the polymer precursor composition is a polyurethane precursor composition, and the polymerizable monomer comprises a polyol and a polyisocyanate. Examples of the polyol include, but are not limited to, ethylene glycol, 1,2-propanediol, 1,3-propanediol, 1,2-butanediol, 1,3-butanediol, 1,4-butanediol, glycerol, trimethylolpropane, pentaerythritol, and ring-opening products of lactones. The foregoing polyols may be used individually or in any combination thereof. Examples of the polyisocyanate include, but are not limited to, methylene diphenyl diisocyanate (MDI), toluene diisocyanate (TDI), hexamethylene diisocyanate (HDI), cyclohexane diisocyanate (CHDI), tetramethyl xylene diisocyanate (TMXDI), 1,3-bis(isocyanatomethyl)cyclohexane (H₆XDI), isophorone diisocyanate (IPDI), and methylene bis(4-cyclohexyl isocyanate) (HMDI). The foregoing polyisocyanates may be used individually or in any combination thereof.

In the polymer precursor composition of the present invention, the content of the polymerizable monomer and the substance of Formula I is not particularly limited, and may be adjusted depending on the desired properties of the polymer material, the type of the polymerizable monomer used, and the desired ultraviolet resistance effect. For example, in embodiments where the above polymer precursor composition is a polyurethane precursor composition, based on the total weight of the polymerizable monomer (comprising the polyol and the polyisocyanate) and the substance of Formula I, the content of the substance of Formula I may be from 0.1 wt% to 30 wt%, more specifically from 0.5 wt% to 3 wt%, for example, 0.1 wt%, 0.5 wt%, 1 wt%, 1.5 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt%, 10.5 wt%, 11 wt%, 11.5 wt%, 12 wt%, 12.5 wt%, 13 wt%, 13.5 wt%, 14 wt%, 14.5 wt%, 15 wt%, 15.5 wt%, 16 wt%, 16.5 wt%, 17 wt%, 17.5 wt%, 18 wt%, 18.5 wt%, 19 wt%, 19.5 wt%, 20 wt%, 20.5 wt%, 21 wt%, 21.5 wt%, 22 wt%, 22.5 wt%, 23 wt%, 23.5 wt%, 24 wt%, 24.5 wt%, 25 wt%, 25.5 wt%, 26 wt%, 26.5 wt%, 27 wt%, 27.5 wt%, 28 wt%, 28.5 wt%, 29 wt%, 29.5 wt%, or 30 wt%, or a range formed between any two of the foregoing values; however, the present invention is not limited thereto.

Without departing from the technical principles of the present invention, the polymer precursor composition of the present invention may further optionally comprise selected components as needed, so as to improve the processability of the polymer precursor composition during manufacturing, promote the polymerization reaction, or tailor the properties of the resulting polymer material. Examples of the foregoing optional components include, but are not limited to, solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, and biocides. These optional components may be used individually or in any combination thereof.

### 2.3. Polymer

The present invention also provides a polymer comprising the substance represented by Formula I, or comprising a structure derived from the substance represented by Formula I.

The type of the polymer of the present invention is not particularly limited, and examples thereof include, but are not limited to, polyurethane, polyester, polycarbonate, epoxy resin, amino resin, polyamide, polyimide, liquid crystal polymer, polyoxymethylene, polysiloxane, polymethacrylate copolymer, polyacrylate copolymer, or composites thereof.

The method for preparing the polymer of the present invention is not particularly limited. For example, the polymer may be obtained by reacting the aforementioned polymer precursor composition through melt polymerization or solution polymerization. Those skilled in the art to which the present invention pertains can prepare the polymer using known methods based on the disclosure of the present specification. In the following examples, the preparation of polyurethane is illustrated for exemplary purposes, and therefore will not be described in further detail herein.

### 2.4. Article Capable of Resisting Ultraviolet Light

The present invention also provides an article, wherein the substance represented by Formula I is used as an ultraviolet light absorber, and thus the article is capable of resisting ultraviolet light. The article may further comprise other conventional ultraviolet light absorbers.

The type of the article capable of resisting ultraviolet light of the present invention is not particularly limited, and may be any article desired to have ultraviolet light resistance. Examples of the article include, but are not limited to, plastics, coatings, inks, displays, lighting devices, optical films, optical lenses, goggles, eyeglasses, contact lenses, textiles, pressure-sensitive adhesives, and sunscreen products.

The article capable of resisting ultraviolet light may further optionally comprise conventional additives as needed so as to tailor the properties of the article. Examples of the foregoing additives include, but are not limited to, solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, and biocides. These optional components may be used individually or in any combination thereof.

### 2.5. Method for Preparing an Article Capable of Resisting Ultraviolet Light

The present invention also provides a method for preparing an article capable of resisting ultraviolet light, characterized in that the substance represented by Formula I is used during the preparation of the article so as to provide ultraviolet resistance. Taking a polymer material as an example, the substance represented by Formula I may be added during the preparation process as a reactive monomer, and the resulting polymer material may be used as all of the constituent materials of the article, or only a portion of the constituent materials (for example, only the surface layer of the article), thereby imparting ultraviolet resistance to the article.

### 3. Method for Preparing the Substance of Formula I

The substance represented by Formula I of the present invention can be prepared by the following method. A compound represented by Formula II is reacted with a C₃ to C₆ lactone compound in the presence of a ring-opening polymerization catalyst, wherein R1 is H or Cl.

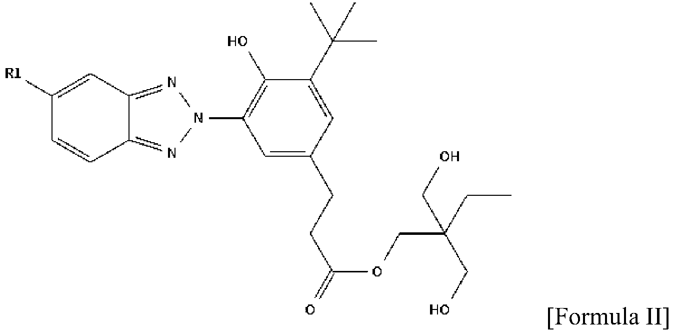

The ring-opening polymerization catalyst refers to a catalyst capable of catalyzing a ring-opening polymerization reaction between the C₃ to C₆ lactone compound and hydroxyl groups. In some embodiments of the present invention, the ring-opening polymerization catalyst is diphenyl phosphate.

The C₃ to C₆ lactone compound refers to a compound having 3 to 6 carbon atoms and an ester group within a cyclic structure. Examples of the C₃ to C₆ lactone compound include β-propiolactone, γ-butyrolactone, δ-valerolactone, and ε-caprolactone, and these compounds may be used individually or in any combination thereof.

The reaction conditions for the ring-opening polymerization reaction depend on the reactants used. Taking diphenyl phosphate as the ring-opening polymerization catalyst and ε-caprolactone as the C₃ to C₆ lactone compound as an example, the ring-opening polymerization reaction may be carried out at a temperature of 25°C to 45°C for 40 minutes to 6 hours. After completion of the reaction, the reaction mixture may be extracted with a solvent, followed by concentration and drying of the extract. The detailed preparation methods are illustrated in the following examples.

### 4. Examples

The present invention is further illustrated in detail by the following specific embodiments.

### 4.1. Preparation of the Substance of Formula I

### [Synthesis Example 1]

A 1 L three-neck round-bottom flask was charged sequentially, at room temperature, with 50 g of the compound represented by Formula IIa (purchased from Chitec Technology Co., Ltd.), 188 g of ε-caprolactone (CL), and 2.75 g of diphenyl phosphate (DPP) as a ring-opening polymerization catalyst, followed by stirring until homogeneous. Thereafter, the reaction mixture was maintained at 30°C for 5 hours to carry out the reaction. After completion of the reaction was confirmed by high-performance liquid chromatography (HPLC), 500 g of toluene and 50 g of a 2% aqueous sodium bicarbonate solution were added for extraction. The organic layer obtained from extraction was concentrated to dryness to obtain the substance represented by Formula Ia, with a conversion rate of 99%. The reaction mechanism is shown below.

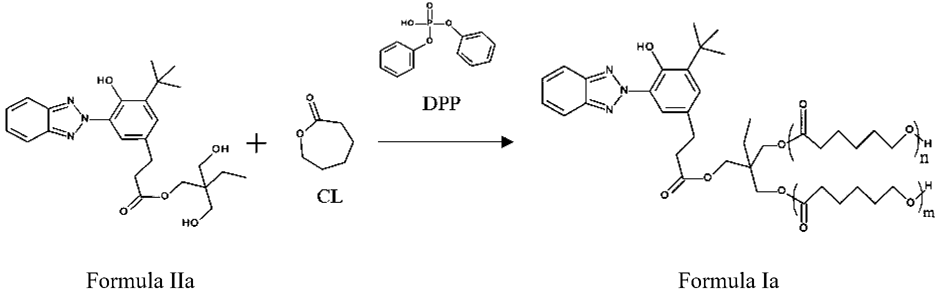

Further analysis of the substance represented by Formula Ia was performed, and the results are as follows:
(1) Gel permeation chromatography (GPC, Model No. Waters 1515):
   The molecular weight was determined to be 2219. Based on calculation, m+n was 16.
(2) Nuclear magnetic resonance spectroscopy (NMR, Model No.VARIAN INOVA 600):
   1H NMR (CDCl₃, 600MHz) δ= 11.78 (s, 1H), 8.09~8.11 (m, 1H), 7.89~7.91 (m, 2H), 7.41~7.46 (m, 2H), 7.13~7.47 (m, 1H), 4.00~4.05 (m, 34H), 3.61 (t, J = 6.0Hz, 4H), 2.92~2.97 (m, 9H), 2.27 (t, J = 6.0Hz, 33H), 1.59~1.63 (m, 65H), 1.30~1.40 (m, 34H), 0.79~0.83 (m, 5H)
(3) Ultraviolet absorption spectrum:
   The absorption spectrum is shown in FIG. 1, with characteristic absorption peaks at 303 nm and 343 nm.

### [Synthesis Example 2]

A 1 L three-neck round-bottom flask was charged sequentially, at room temperature, with 50 g of the compound represented by formula IIa (purchased from Chitec Technology Co., Ltd.), 2508 g of ε-caprolactone, and 55 g of diphenyl phosphate as a ring-opening polymerization catalyst, followed by stirring until homogeneous. Thereafter, the reaction mixture was maintained at 40°C for 2 hours to carry out the reaction. After completion of the reaction was confirmed by high-performance liquid chromatography, 6500 g of toluene and 650 g of a 2% aqueous sodium bicarbonate solution were added for extraction. The organic layer obtained from extraction was concentrated to dryness to obtain the substance represented by Formula Ia, with a conversion rate of 50%. Gel permeation chromatography analysis showed that the molecular weight of the obtained product was 11920. Based on calculation, m+n was 100.

### [Synthesis Example 3]

A 1 L three-neck round-bottom flask was charged sequentially, at room temperature, with 50 g of the compound represented by Formula IIa (purchased from Chitec Technology Co., Ltd.), 940 g of ε-caprolactone, and 55 g of diphenyl phosphate as a ring-opening polymerization catalyst, followed by stirring until homogeneous. Thereafter, the reaction mixture was maintained at 40°C for 1 hour to carry out the reaction. After completion of the reaction was confirmed by high-performance liquid chromatography, 1100 g of toluene and 110 g of a 2% aqueous sodium bicarbonate solution were added for extraction. The organic layer obtained from extraction was concentrated to dryness to obtain the substance represented by Formula Ia, with a conversion rate of 99%. Gel permeation chromatography analysis showed that the molecular weight of the obtained product was 8,990. Based on calculation, m+n was 74.

### [Synthesis Example 4]

A 1 L three-neck round-bottom flask was charged sequentially, at room temperature, with 50 g of the compound represented by Formula IIa (purchased from Chitec Technology Co., Ltd.), 225 g of ε-caprolactone, and 2.75 g of diphenyl phosphate as a ring-opening polymerization catalyst, followed by stirring until homogeneous. Thereafter, the reaction mixture was maintained at 30°C for 3 hours to carry out the reaction. After completion of the reaction was confirmed by high-performance liquid chromatography, 600 g of toluene and 60 g of a 2% aqueous sodium bicarbonate solution were added for extraction. The organic layer obtained from extraction was concentrated to dryness to obtain the substance represented by Formula Ia, with a conversion rate of 80%. Gel permeation chromatography analysis showed that the molecular weight of the obtained product was 2110. Based on calculation, m+n was 28.

### 4.2. Solubility and Dispersibility Test

100 g of polycaprolactone polyol was used as a blank sample and observed at operating temperatures of 25°C, 50°C, and 100°C. In addition, 5 g of the substance of Formula Ia of Synthesis Example 1 or 5 g of the compound of Formula IIa was respectively added to 100 g of polycaprolactone polyol and mixed. The dispersibility was then observed at operating temperatures of 25°C, 50°C, and 100°C, and the results are summarized in Table 1.

**Table 1: Solubility and dispersibility of the substance of Formula Ia of Synthesis Example 1 and the compound of Formula IIa in polycaprolactone polyol**

| Operating temperature | Polycaprolactone polyol | Polycaprolactone polyol + substance of Formula Ia of Synthesis Example 1 | Polycaprolactone polyol + compound of Formula IIa |
|---|---|---|---|
| 25°C | waxy solid | waxy solid | waxy solid |
| 50°C | transparent liquid | transparent liquid | liquid containing suspended particles |
| 100°C | transparent liquid | transparent liquid | transparent liquid |

As shown in Table 1, polycaprolactone polyol is a waxy solid at 25°C and becomes a transparent liquid when heated to 50°C. Table 1 further shows that the polycaprolactone polyol containing the compound of Formula IIa is a liquid with suspended particles at 50°C, and becomes a transparent liquid only when heated to 100°C, indicating that the compound of Formula IIa can be uniformly dissolved and dispersed in the polycaprolactone polyol only at 100°C. In contrast, the polycaprolactone polyol containing the substance of Formula Ia of Synthesis Example 1 of the present invention becomes a transparent liquid at 50°C, indicating that the substance of Formula Ia is already uniformly dispersed in the polycaprolactone polyol at 50°C. These results fully demonstrate that the substance represented by Formula Ia of the present invention has the advantage of being operable at low temperatures.

### 4.3. Preparation of Polymer

### [Example 1]

110 g of polycaprolactone polyol (Model: PCL-3000(230N), purchased from Daicel Chemical Industries, Japan), 15 g of 1,4-butanediol (purchased from Tokyo Chemical Industry Co., Ltd.), 0.2 wt% (based on the total weight of the reactants) of a hindered amine light stabilizer (Model: Chiguard 106, purchased from Chitec Technology Co., Ltd.), 0.7 wt% (based on the total weight of the reactants) of an antioxidant (Model: Revonox 5068L, purchased from Chitec Technology Co., Ltd.), and 4.3 g of the substance of Formula Ia of Synthesis Example 1 were charged into a reaction iron vessel, followed by heating to 50°C and mixing until homogeneous. Thereafter, 20 ppm of dibutyltin dilaurate was added into the reaction iron vessel and mixed uniformly, followed by heating to 110°C. Separately, 52 g of methylene diphenyl diisocyanate (MDI) (purchased from BASF) was preheated to 110°C and then added into the reaction iron vessel, followed by stirring for 1 minute to carry out the reaction and obtain a rubber block. Subsequently, the rubber block was placed in an oven and cured at 70°C for 24 hours to obtain a thermoplastic polyurethane rubber block of Example 1, wherein the content of the substance of formula Ia was 0.5 wt%.

### [Example 2]

A thermoplastic polyurethane was prepared in the same manner as in Example 1, except that the amount of the substance of Formula Ia of Synthesis Example 1 was adjusted to 8.6 g, thereby obtaining a thermoplastic polyurethane rubber block of Example 2, wherein the content of the substance of formula Ia was 1.0 wt%.

### [Example 3]

A thermoplastic polyurethane was prepared in the same manner as in Example 1, except that the amount of the polycaprolactone polyol was adjusted to 84 g and the amount of the substance of formula Ia of Synthesis Example 1 was adjusted to 22 g, thereby obtaining a thermoplastic polyurethane rubber block of Example 3, wherein the content of the substance of Formula Ia was 3.0 wt%.

### [Comparative Example 1]

A thermoplastic polyurethane was prepared in the same manner as in Example 1, except that the amount of the polycaprolactone polyol was adjusted to 120 g and the substance of Formula Ia of Synthesis Example 1 was not used, thereby obtaining a thermoplastic polyurethane rubber block of Comparative Example 1.

### 4.4. Property Testing of Polymers

### 4.4.1. Preparation of Thermoplastic Polyurethane Specimens

Thermoplastic polyurethane specimens were prepared using the thermoplastic polyurethane rubber blocks of Examples 1 to 3 and Comparative Example 1, respectively. First, 55 g of the thermoplastic polyurethane rubber block was placed in an oven at 100°C for 2 hours to remove surface moisture and achieve sufficient drying. Subsequently, the dried thermoplastic polyurethane rubber block was placed into a mold measuring 20 cm × 15 cm × 0.15 cm. Hot pressing was performed using a hot press molding machine (purchased from Longchang Co., Ltd.) at 20 kg/cm² and 190°C for 1.5 minutes. The hot-pressed thermoplastic polyurethane was then transferred to a cold press machine and cooled under a pressure of 50 kg/cm² for 5 to 10 minutes, thereby obtaining thermoplastic polyurethane specimens having a thickness of 0.15 cm.

### 4.4.2. Test Instruments and Methods

### [Melt Flow Index (MFI) Measurement]

5 g of thermoplastic polyurethane was prepared as a test sample. According to ASTM D1238, the initial melt flow index (MFI) of the thermoplastic polyurethane was measured using a melt flow index tester (Model: GT-7100-MI, purchased from GOTECH) under the following conditions: a temperature of 200°C and a load of 5 kg, measuring the weight of material extruded through a standard die (diameter: 2.095 mm) within 10 minutes. The melt flow index (MFI) is expressed in grams per 10 minutes (g/10 min).

### [Hue Measurement]

According to ASTM D1926-70, the initial yellow index (YI) of the thermoplastic polyurethane specimens was measured using a spectrophotometric color difference meter (Model: ColorQuest XE, purchased from HunterLab).

### [Color Difference Measurement]

The ΔE value of the thermoplastic polyurethane specimens was measured using a UV-Vis spectrophotometer (Model: Varian Cary^{®} 50, purchased from Agilent).

### [Light Aging Yellowing Test (QUV340 Test)]

According to ISO 11341, the thermoplastic polyurethane specimens were exposed to ultraviolet irradiation for 888 hours in an accelerated weathering tester using a xenon lamp. The ΔYI value and ΔE value of the thermoplastic polyurethane specimens were measured after 72, 240, 408, 600, and 888 hours of irradiation, respectively. Lower values indicate better resistance to light aging-induced yellowing.

### 4.4.3. Test Results

The properties of the thermoplastic polyurethanes of Examples 1 to 3 and Comparative Example 1 were measured according to the aforementioned measurement methods, including melt flow index (MFI), initial yellow index (YI), and ΔYI and ΔE after ultraviolet irradiation. The results are summarized in Table 2.

**Table 2: Properties of thermoplastic polyurethanes of Examples 1 to 3 and Comparative Example 1**

| | | Example 1 | Example 2 | Example 3 | Comparative Example 1 |
|---|---|---|---|---|---|
| Amount of the substance of Formula Ia | | 0.5 wt% | 1.0 wt% | 3.0 wt% | - |
| MFI | | 2.0 | 5.6 | 1.0 | 4.4 |
| Initial YI | | 11.7 | 9.9 | 14.8 | 3.2 |

| Light aging yellowing test (QUV340 test) | | | | | |
|---|---|---|---|---|---|
| ΔYI after ultraviolet irradiation | 72 hours | 7.3 | 3.7 | 0.7 | 25.4 |
| | 240 hours | 23.7 | 14.2 | 5.5 | 44.3 |
| | 408 hours | 33.6 | 23.5 | 11.9 | 49.7 |
| | 600 hours | 37.6 | 29.4 | 17.2 | 50.8 |
| | 888 hours | 43.4 | 34.1 | 23.4 | 70.7 |
| ΔE after ultraviolet irradiation | 72 hours | 4.3 | 2.2 | 0.4 | 15.3 |
| | 240 hours | 14.4 | 8.5 | 3.2 | 27.6 |
| | 408 hours | 20.6 | 14.3 | 7.1 | 31.1 |
| | 600 hours | 23.2 | 18.0 | 10.4 | 31.9 |
| | 888 hours | 26.8 | 20.9 | 14.2 | 45.8 |

As shown in Table 2, Comparative Example 1, in which the substance of formula Ia of the present invention was not used as an ultraviolet light absorber, exhibited poor performance in the light aging yellowing test of the resulting thermoplastic polyurethane. In contrast, the thermoplastic polyurethanes of Examples 1 to 3, which used the substance of formula Ia of the present invention as an ultraviolet light absorber, exhibited excellent resistance to light aging. Specifically, Example 1 demonstrates that incorporating only 0.5 wt% of the substance of formula Ia in the preparation of thermoplastic polyurethane can significantly reduce the ΔYI and ΔE values in the light aging yellowing test, indicating that the present invention indeed provides thermoplastic polyurethane with improved resistance to light aging-induced yellowing. In addition, Examples 1 to 3 show that as the content of the substance of formula Ia increases from 0.5 wt% to 3 wt%, the resulting thermoplastic polyurethane exhibits further improved ΔYI and ΔE performance.

The above examples are used to illustrate the principle and efficacy of the present invention and show the inventive features thereof, but are not used to limit the scope of the present invention. Any changes or modifications that can be readily made by a person skilled in the art without departing from the technical principles of the present invention shall fall within the scope of the present invention as claimed.

## Claims

1. A substance represented by Formula I, 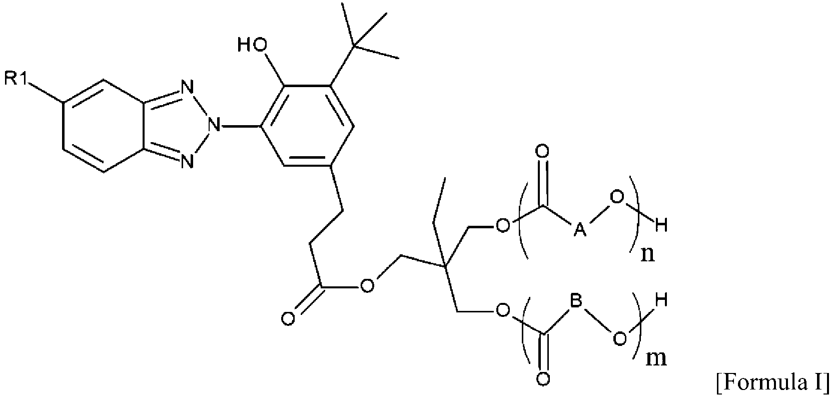 wherein,
R1 is H or Cl;
A is C₂ to C₅ alkylene;
B is C₂ to C₅ alkylene; and
m+n is an integer from 2 to 120, with neither m nor n being 0.

2. The substance of claim 1, wherein each of A and B is independently C₅ alkylene.

3. The substance of claim 1, wherein each of m and n is independently an integer from 1 to 50.

4. Use of the substance of any one of claims 1 to 3, wherein the substance is used as an ultraviolet light absorber.

5. A polymer precursor composition, comprising:
a polymerizable monomer; and the substance of any one of claims 1 to 3.

6. The polymer precursor composition of claim 5, wherein the polymerizable monomer comprises a polyol and a polyisocyanate.

7. The polymer precursor composition of claim 5, which further comprises at least one additive selected from the group consisting of solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, biocides, and combinations thereof.

8. A polymer, comprising a structure derived from the substance of any one of claims 1 to 3.

9. The polymer of claim 8, which is selected from the group consisting of polyurethane, polyester, polycarbonate, epoxy resin, amino resin, polyamide, polyimide, liquid crystal polymer, polyoxymethylene, polysiloxane, polymethacrylate copolymer, polyacrylate copolymer, and composites thereof.

10. An article capable of resisting ultraviolet light, which applies the substance of any one of claims 1 to 3 as an ultraviolet light absorber.

11. The article of claim 10, which is selected from the group consisting of plastics, coatings, inks, displays, lighting devices, optical films, optical lenses, goggles, eyeglasses, contact lenses, textiles, pressure-sensitive adhesives, and sunscreen products.

12. The article of claim 10, where the article further comprises at least one additive selected from the group consisting of solvents, catalysts, antioxidants, fillers, compatibilizers, flame retardants, thermal stabilizers, light stabilizers, metal deactivators, plasticizers, lubricants, emulsifiers, dyes, pigments, brighteners, antistatic agents, blowing agents, chain extenders, hydrolysis stabilizers, surfactants, crosslinking agents, photoinitiators, pH regulators, adhesion promoters, biocides, and combinations thereof.

13. A method for preparing an article capable of resisting ultraviolet light, comprising using the substance of any one of claims 1 to 3 in the article.

14. A method for preparing the substance of any one of claims 1 to 3, comprising reacting a compound represented by Formula II with a C₃ to C₆ lactone compound in the presence of a ring-opening polymerization catalyst, 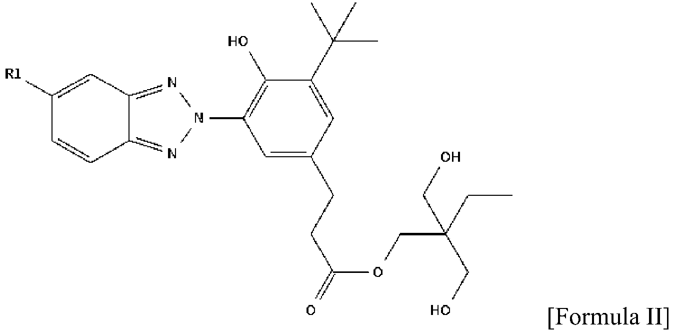 wherein R1 is H or Cl.

15. The method of claim 14, wherein the ring-opening polymerization catalyst is diphenyl phosphate.

16. The method of claim 14, wherein the C₃ to C₆ lactone compound is selected from the group consisting of β-propiolactone, γ-butyrolactone, δ-valerolactone, ε-caprolactone, and combinations thereof.
